# EUROPEAN PATENT SPECIFICATION

(11) **EP 3 785 299 B1**
(45) Date of publication and mention of the grant of the patent: **01.06.2022**
(21) Application number: 19820427.3
(22) Date of filing: 10.06.2019
(51) Int. Cl.: F21S 43/14, B60Q 1/00, B60Q 1/26, B60Q 1/50

(54) **VEHICLE LIGHT WITH 3-DIMENSIONAL APPEARANCE**
FAHRZEUGLEUCHTE MIT DREIDIMENSIONALEM ERSCHEINUNGSBILD
FEU DE VÉHICULE AYANT UN ASPECT TRIDIMENSIONNEL

(30) Priority: 14.06.2018 US 201862684812 P
(43) Date of publication of application: 03.03.2021
(73) Proprietor: Magna International Inc., Aurora, ON L4G 7K1 (CA)
(72) Inventor: SALTSMAN, Benjamin, Bloomfield Township, MI 48301 (US)
(74) Representative: Glawe, Delfs, Moll
(86) International application number: PCT/US2019/036266
(87) International publication number: WO 2019/241101

(56) References cited:
- EP-A1- 2 295 849
- EP-A2- 0 950 848
- DE-A1-102016 107 089
- US-A1- 2004 170 014
- US-A1- 2012 268 266
- US-A1- 2013 107 525
- US-A1- 2014 096 893
- US-A1- 2014 098 517
- US-A1- 2015 003 083
- US-A1- 2015 167 918
- US-A1- 2016 154 170
- US-A1- 2017 176 664
- US-B1- 6 848 819

## Description

### CROSS-REFERENCE TO RELATED APPLICATIONS

This PCT International Patent Application claims the benefit of U.S. Provisional Patent Application Serial No. 62/684,812 filed on June 14, 2018, and titled "Vehicle Light With 3-Dimensional Appearance".

### FIELD

The present invention relates generally to lighting devices for vehicles.

### BACKGROUND

Traditional lighting and in particular automotive light, such as tail lamps is done with incandescent bulbs, with discrete LEDs or OLED. Discrete (packaged LEDs) most commonly used today provide a pixilated appearance and may require complex diffuser materials to smooth out and to blend the emitted light pattern. OLED tail lamps provide a desired uniform appearance, but are extremely expensive and suffer from long term durability performance issues. US 2014/096893 A1 proposes a method of making a windshield for a vehicle, comprising providing a deformable backbone supporting a plurality of LED devices, wherein the backbone is laminated between first and second glass substrates to have a shape selected to match a certain angle. US 2016/154170 A1 proposes various applications and customizations of a thin flexible LED light sheet. US 2015/167918 A1 proposes a light source module for a vehicle, including an LED light source and a patterned film provided with an optical pattern which enables a stereoscopic light-emission image of a desired pattern to be formed when light from the LED light source is emitted thereto.

Optical visual art with 3D patterns was first introduced by artists such as Victor Vasarely in the 1930's. In his art, 2D drawings appear 3D due to purposeful bending or distortion of lines.

### SUMMARY

A lighting device for a vehicle comprising a plurality of MicroLED dies disposed in a continuous surface of a printed circuit board or a flexible circuit is provided. The plurality of MicroLED dies are configured to present an appearance of a 3-dimensional object such that the lighting device provides an accentuated 3-dimensional visual appearance from a surface that is substantially flat, and each of the Micro LED dies within the lighting device is individually or group addressable by a controller to illuminate the plurality of Micro LED dies in one or more different patterns to appear as the 3-dimensional object.

### BRIEF DESCRIPTION OF THE DRAWINGS

Further details, features and advantages of designs of the invention result from the following description of embodiment examples in reference to the associated drawings.
FIG. 1 is a profile view of a lighting device of the present invention;
FIG. 2 is a block diagram of the lighting device of the present invention and
FIGS. 3A, 3B, 3C and 3D show a lighting device of the present invention at different stages of a pattern.

### DETAILED DESCRIPTION

Recurring features are marked with identical reference numerals in the figures, in which an example embodiment of a lighting device 20 for a vehicle and presenting a 3-dimensional object 22 is disclosed. Specifically, the lighting device 20 provides an accentuated 3-dimensional visual appearance from a surface that is substantially flat. The lighting device **20** includes a plurality of very small light emitting diodes (MicroLED dies) disposed in a continuous surface of a printed circuit board or a flexible substrate such as polyimide (PI) with copper traces or polyester (PET) with silver traces. The continuous surface lies entirely or substantially within a two-dimensional plane. The accentuated 3-dimensional visual appearance of the 3-dimensional (3D) object **22** may appear to have depth or height that is substantially greater than a thickness of the continuous surface.

A purpose of this invention is to leverage capabilities afforded by multitude of small LEDs placed in close proximity to each other and having capability to be individually addressed (meaning each LED can be individually turned on and off and intensity controlled) or in small groups or in lines on the circuit. The lighting device **20** presents a 3-dimensional object which can be animated, making the pattern pulse and creating the appearance of a bulging out of the plane. The lighting device **20** creates a visual 3D impression while maintaining a substantially flat thin LED package.

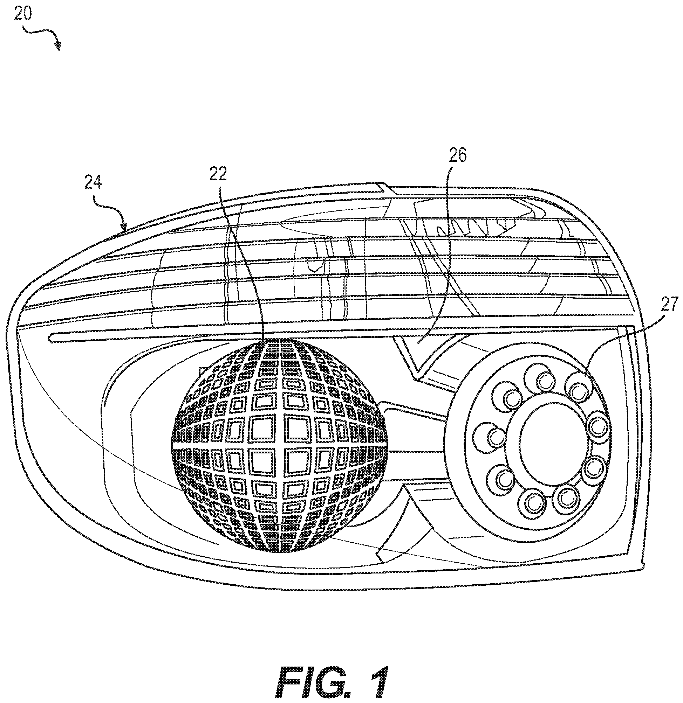

As shown in FIG. 1, an example application of the subject lighting device **20** is in a brake light assembly **24** for a vehicle, where it is located behind a plastic lens **26.** However, the subject lighting device **20** may be used in other applications such as in a turn signal indicator or a reverse light. In some embodiments, the lighting device **20** may include one or more discrete illumination sources **27,** such as incandescent bulbs or individual light emitting diodes (LEDs), and which is separate and independent from the 3-dimensional object **22.** For example, the brake light assembly **24** of FIG. 1 includes both the 3-dimensional object **22** and a grouping of discrete illumination sources **27** in the form of LEDs. A common signal, such as application of brakes of the vehicle, may activate the discrete illumination sources **27** and also cause the 3-dimensional object **22** to be illuminated and/or animated.

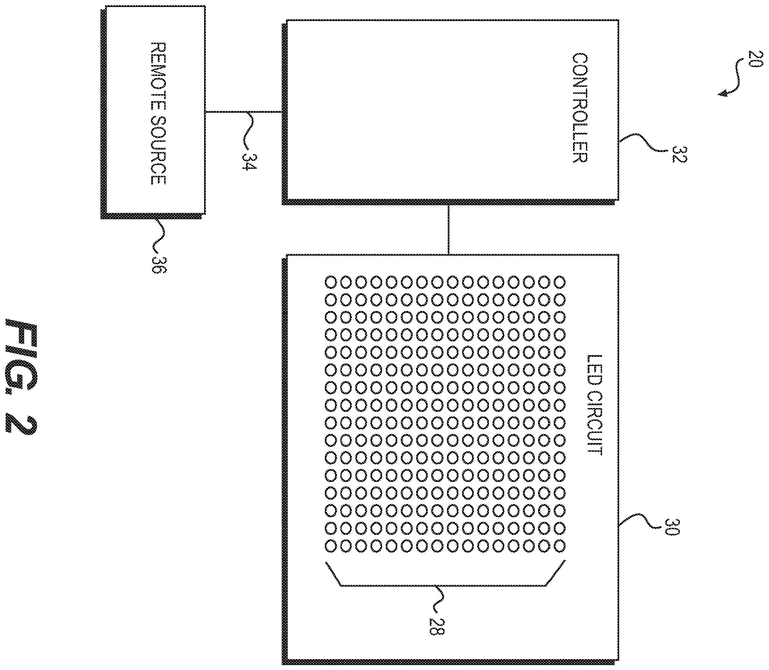

As shown in FIG. 2, the lighting device **20** includes a plurality of MicroLED dies **28,** which are very small discrete light emitting diodes, each measuring approximately 0.2 mm x 0.2 mm to about 0.2 mm x 0.3 mm. The MicroLED dies **28** are disposed in a continuous surface of a printed circuit board **30** or a flexible substrate, such as, for example, polyimide (PI) with copper traces or polyester (PET) with silver traces. The lighting device **20** has a center-to-center pitch between two adjacent ones of the MicroLED dies **28** that is greater than or equal to 0.3mm. In some embodiments, the lighting device **20** has a center-to-center pitch between adjacent MicroLED dies **28** of 1-6 mm. In some embodiments, a diffuser layer may overlie the plurality of MicroLED dies **28.** The diffuser layer may provide a more uniform light appearance, particularly where the MicroLED dies **28** have a relatively large pitch. The MicroLED dies **28** may be arranged in horizontal and vertical rows as shown, although they may form other patterns such as, but not limited to, offset rows, one or more circular patterns, diamond patterns, etc. Because the subject lighting device **20** includes MicroLED dies **28** on a printed circuit board or a flexible substrate **30,** it may have a thinner package size and lighter weight when compared to existing lighting devices that use incandescent bulbs or traditional LEDs, and which include relatively large and heavy structural elements such as reflectors and lenses to provide a 3-dimensional appearance.

In some embodiments, the printed circuit board 30 or flexible substrate may be separate and independent from any discrete illumination sources 27 within the lighting device 20. Alternatively, one or more discrete illumination sources 27 may be disposed upon and/or controlled by the printed circuit board 30 or flexible substrate. In some embodiments, the MicroLED dies 28 may overlie one or more of the discrete illumination sources 27. Alternatively or additionally, the MicroLED dies 28 may be located separately from one or more of the discrete illumination sources 27 so the both the 3-dimensional object 22 the discrete illumination sources 27 can be separately visible. One such arrangement is shown on FIG. 1.

According to the invention, each of the MicroLED dies **28** within the lighting device **20** is individually or group addressable by a controller **32.** In some embodiments, each of the MicroLED dies **28** can be individually controlled via a command by the controller **32.** Each of the MicroLED dies **28** is configured to have a brightness and/or a color that is adjustable in response to a command by the controller **32.** The brightness may vary from black (i.e. totally off) to a maximum intensity. In some embodiments, a group of two or more of the MicroLED dies **28** can be controlled via a command by the controller **32.** For example, the controller **32** may command a group of two or more of the MicroLED dies **28** to all have a specified color or brightness setting. In another example, the controller **32** may command a group of two or more of the MicroLED dies **28** to illuminate with a predetermined pattern, setting or animation. In other words, individual ones of the MicroLED dies **28** within a group may have a different brightness and/or color at any given time.

The controller **32** is configured to illuminate the plurality of MicroLED dies **28** in one or more different patterns to appear as the 3-dimensional object **22.** The patterns appearing as the 3-dimensional object **22** may, for example, be similar to those shown in graphic artworks from the Op Art movement, which employ optical illusions to give the appearance of depth, movement, hidden images, flashing, vibrating, warping, swelling, or oscillating patterns. In some embodiments, the 3-dimensional object **22** may appear to swell and to increase in size over time. Examples of such Op Art artworks include works by Victor Vasarely.

The controller **32** may be configured to illuminate the plurality of MicroLED dies **28** in response to a control signal **34** from a remote source **36,** such as a switch or another computerized controller, such as a body control module (BCM) within the vehicle. The control signal may, for example, correspond to a turn signal or a braking condition or a reversing condition of the vehicle or other messaging or signaling illumination displayed using available tail lamp or light or another lighting circuit of the vehicle. Other messaging / signaling illumination may include, for example, emergency hazard lights, running lights, confirmation of locking or unlocking status, or annunciation of an alarm condition.

According to a further aspect, the controller **32** is configured to animate the 3-dimensional object **22** in a time-varying pattern, which may, for example, make the 3-dimensional object **22** appear to move into or out of a plane which is on or parallel to the printed circuit board or flexible substrate **30.** In other words, the 3-dimensional object **22** may appear to protrude outwardly from the plane within the brake light assembly **24.** The 3-dimensional object **22** may appear to come out beyond the plastic lens **26.** The 3-dimensional object **22** may be animated in a pulsing pattern, which can help to make it more interesting or to enhance visibility of the associated signaling condition.

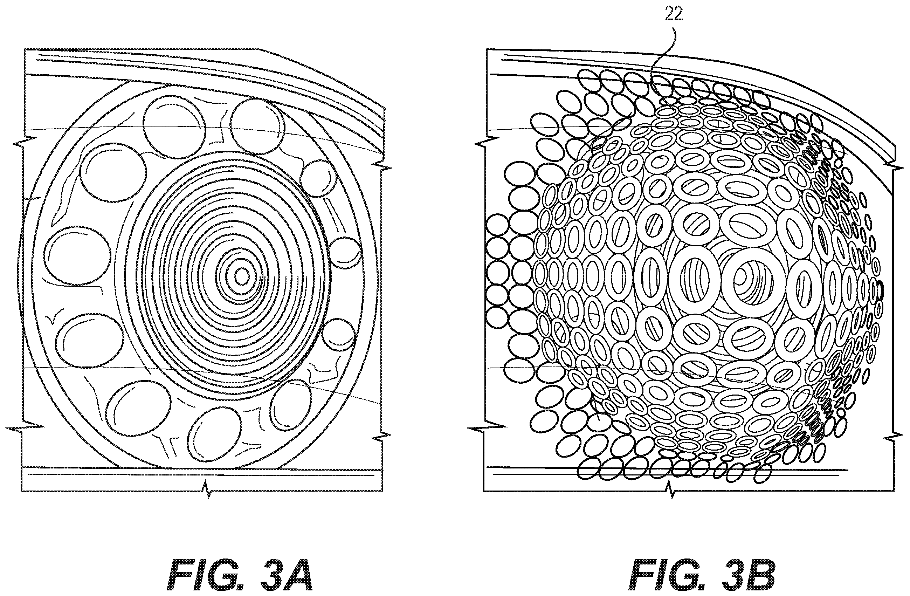

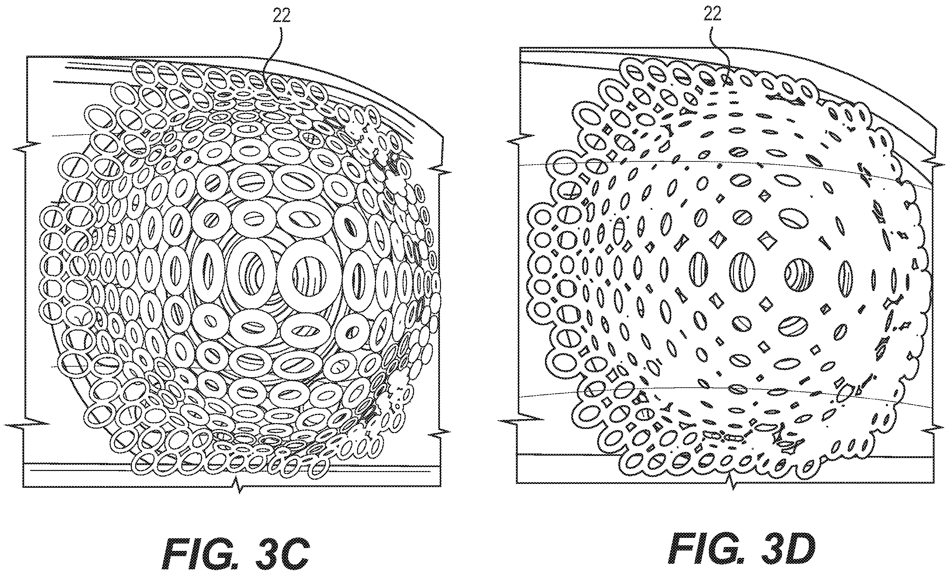

An example of the lighting device **20** with such a time-varying pattern is shown in FIGS. 3A - 3D, in which a 3-dimensional object **22** appears and which gets progressively larger and/or brighter over time. The 3-dimensional object **22** may also get progressively smaller and/or dimmer over time, reversing the steps illustrated in FIGS. 3A-3D.

The system, methods and/or processes described above, and steps thereof, may be realized in hardware, software or any combination of hardware and software suitable for a particular application. The hardware may include a general purpose computer and/or dedicated computing device or specific computing device or particular aspect or component of a specific computing device. The processes may be realized in one or more microprocessors, microcontrollers, embedded microcontrollers, programmable digital signal processors or other programmable device, along with internal and/or external memory. The processes may also, or alternatively, be embodied in an application specific integrated circuit, a programmable gate array, programmable array logic, or any other device or combination of devices that may be configured to process electronic signals. It will further be appreciated that one or more of the processes may be realized as a computer executable code capable of being executed on a machine readable medium.

The computer executable code may be created using a structured programming language such as C, an object oriented programming language such as C++, or any other high-level or low-level programming language (including assembly languages, hardware description languages, and database programming languages and technologies) that may be stored, compiled or interpreted to run on one of the above devices as well as heterogeneous combinations of processors processor architectures, or combinations of different hardware and software, or any other machine capable of executing program instructions.

Thus, in one aspect, each method described above and combinations thereof may be embodied in computer executable code that, when executing on one or more computing devices performs the steps thereof. In another aspect, the methods may be embodied in systems that perform the steps thereof, and may be distributed across devices in a number of ways, or all of the functionality may be integrated into a dedicated, standalone device or other hardware. In another aspect, the means for performing the steps associated with the processes described above may include any of the hardware and/or software described above.

The foregoing description of the embodiments has been provided for purposes of illustration and description. It is not intended to be exhaustive or to limit the invention as defined by the claims.

## Claims

1. A lighting device (20) for a vehicle comprising:
a plurality of MicroLED dies (28) disposed on a continuous surface of a printed circuit board (30) or a flexible circuit; **characterised in that**
the plurality of MicroLED dies (28) are configured to present an appearance of a 3-dimensional object (22) such that the lighting device (20) provides an accentuated 3-dimensional visual appearance from a surface that is substantially flat, and each of the MicroLED dies (28) within the lighting device (20) is individually or group addressable by a controller (32) to illuminate the plurality of MicroLED dies (28) in one or more different patterns to appear as the 3-dimensional object (22).

2. The lighting device (20) as set forth in Claim 1, wherein each of the MicroLED dies (28) measures 0.2 mm x 0.2 mm.

3. The lighting device (20) as set forth in Claim 1, wherein the plurality of MicroLED dies (28) defines a center-to-center pitch of greater than 0.3 mm between adjacent MicroLED dies (28) within the plurality of MicroLED dies (28).

4. The lighting device (20) as set forth in Claim 1, wherein the plurality of MicroLED dies (28) defines a center-to-center pitch of 1 mm to 6 mm between adjacent MicroLED dies (28) within the plurality of MicroLED dies (28).

5. The lighting device (20) as set forth in Claim 1, further comprising a diffuser layer overlying the plurality of MicroLED dies (28).

6. The lighting device (20) as set forth in Claim 1, wherein each MicroLED die within the plurality of MicroLED dies (28) is individually addressable by the controller (32);
wherein each MicroLED die within the plurality of MicroLED dies (28) is configured to have at least one of a brightness or a color that is adjustable in response to a command by the controller (32); and
wherein the controller (32) is configured to illuminate the plurality of MicroLED dies (28) to appear as the 3-dimensional object (22).

7. The lighting device (20) as set forth in Claim 1,
wherein each MicroLED die within the plurality of MicroLED dies (28) is configured to have at least one of a brightness or a color that is adjustable in response to a command by the controller (32).

8. The lighting device (20) as set forth in Claim 1, wherein the controller (32) is configured to cause the plurality of MicroLED (28) dies to illuminate in response to a control signal (34).

9. The lighting device (20) as set forth in Claim 8, wherein the control signal (34) corresponds to one of a turn signal or a braking condition or a reversing condition of the vehicle or a messaging or signaling illumination displayed using a tail lamp or a headlight.

10. The lighting device (20) as set forth in Claim 1, wherein the 3-dimensional object is animated with a time-varying pattern.

11. The lighting device (20) as set forth in Claim 10, wherein the time-varying pattern makes the 3-dimensional object (22) appear to move into a plane on or parallel to the printed circuit board (30).

12. The lighting device as set forth in Claim 10, wherein the time-varying pattern makes the 3-dimensional object appear to move out of a plane on or parallel to the printed circuit board.

13. The lighting device (20) as set forth in Claim 10, wherein the time-varying pattern makes the 3-dimensional object (22) appear to increase in size over time.

14. The lighting device (20) as set forth in Claim 10, wherein the 3-dimensional object (22) is animated with a pulsing pattern.

15. The lighting device (20) as set forth in Claim 1, further comprising: a discrete illumination source (27).

## Patentansprüche

1. Beleuchtungsvorrichtung (20) für ein Fahrzeug, die aufweist:
eine Anzahl von Mikro-LED-Dioden (28), die auf einer durchgehenden Oberfläche einer Leiterplatte (30) oder einer flexiblen Schaltung angeordnet sind, **dadurch gekennzeichnet, dass** die mehreren Mikro-LED-Dioden (28) so ausgebildet sind, dass sie ein Erscheinungsbild eines dreidimensionalen Objekts (22) präsentieren, so dass die Beleuchtungsvorrichtung (20) ein akzentuiertes dreidimensionales visuelles Erscheinungsbild von einer Oberfläche aus liefert, die im Wesentlichen flach ist, und jede der Mikro-LED-Dioden (28) innerhalb der Beleuchtungsvorrichtung (20) einzeln oder als Gruppe durch einen Controller (32) adressierbar ist, um die mehreren Mikro-LED-Dioden (28) in einem oder mehreren unterschiedlichen Mustern zu beleuchten, um als das dreidimensionale Objekt (22) zu erscheinen.

2. Beleuchtungsvorrichtung (20) nach Anspruch 1, wobei jede der Mikro-LED-Dioden (28) 0,2 mm x 0,2 mm misst.

3. Beleuchtungsvorrichtung (20) nach Anspruch 1, wobei die Anzahl von Mikro-LED-Dioden (28) einen Mittenabstand von mehr als 0,3 mm zwischen benachbarten Mikro-LED-Dioden (28) innerhalb der Anzahl von Mikro-LED-Dioden (28) definiert.

4. Beleuchtungsvorrichtung (20) nach Anspruch 1, wobei die Anzahl von Mikro-LED-Dioden (28) einen Mittenabstand von 1 mm bis 6 mm zwischen benachbarten Mikro-LED-Dioden (28) innerhalb der Anzahl von Mikro-LED-Dioden (28) definiert.

5. Beleuchtungsvorrichtung (20) nach Anspruch 1, ferner mit einer Diffusorschicht, die über der Anzahl von Mikro-LED-Dioden (28) liegt.

6. Beleuchtungsvorrichtung (20) nach Anspruch 1, wobei jedes Mikro-LED-Diode innerhalb der Anzahl von Mikro-LED-Dioden (28) durch den Controller (32) einzeln adressierbar ist,
wobei jede Mikro-LED-Diode innerhalb der Anzahl von Mikro-LED-Dioden (28) so ausgebildet ist, dass sie mindestens eine Helligkeit oder eine Farbe aufweist, die als Reaktion auf einen Befehl durch den Controller (32) einstellbar ist, und
wobei der Controller (32) so ausgebildet ist, dass er die Anzahl von Mikro-LED-Dioden (28) beleuchtet, um als das dreidimensionale Objekt (22) zu erscheinen.

7. Beleuchtungsvorrichtung (20) nach Anspruch 1,
wobei jede Mikro-LED-Diode innerhalb der Anzahl von Mikro-LED-Dioden (28) so ausgebildet ist, dass sie zumindest eine Helligkeit oder eine Farbe aufweist, die als Reaktion auf einen Befehl durch den Controller (32) einstellbar ist.

8. Beleuchtungsvorrichtung (20) nach Anspruch 1, wobei der Controller (32) so ausgebildet ist, dass er als Reaktion auf ein Steuersignal (34) bewirkt, dass die Mehrzahl von Mikro-LED (28)-Dioden leuchtet.

9. Beleuchtungsvorrichtung (20) nach Anspruch 8, wobei das Steuersignal (34) einem Blinker oder einem Bremszustand oder einem Rückwärtsfahrzustand des Fahrzeugs oder einer Nachrichten- oder Signalbeleuchtung entspricht, die unter Verwendung eines Rücklichts oder eines Scheinwerfers angezeigt wird.

10. Beleuchtungsvorrichtung (20) nach Anspruch 1, wobei das dreidimensionale Objekt mit einem zeitlich veränderlichen Muster animiert ist.

11. Beleuchtungsvorrichtung (20) nach Anspruch 10, wobei das zeitlich veränderliche Muster das dreidimensionale Objekt (22) in eine Ebene auf oder parallel zu der Leiterplatte (30) zu bewegen scheint.

12. Beleuchtungsvorrichtung nach Anspruch 10, wobei das zeitlich veränderliche Muster das dreidimensionale Objekt aus einer Ebene auf oder parallel zu der gedruckten Leiterplatte herauszubewegen scheint.

13. Beleuchtungsvorrichtung (20) nach Anspruch 10, wobei das zeitlich veränderliche Muster das dreidimensionale Objekt (22) mit der Zeit größer erscheinen lässt.

14. Beleuchtungsvorrichtung (20) nach Anspruch 10, wobei das dreidimensionale Objekt (22) mit einem pulsierenden Muster animiert ist.

15. Beleuchtungsvorrichtung (20) nach Anspruch 1, ferner umfassend: eine diskrete Beleuchtungsquelle (27).

## Revendications

1. Dispositif d'éclairage (20) pour un véhicule comprenant :
une pluralité de matrices MicroLED (28) disposées sur une surface continue d'une carte à circuit imprimé (30) ou d'un circuit flexible ;
**caractérisé en ce que**
la pluralité de matrices MicroLED (28) sont configurées pour présenter un aspect visuel d'un objet tridimensionnel (22) de telle sorte que le dispositif d'éclairage (20) fournit un aspect visuel tridimensionnel accentué depuis une surface qui est sensiblement plate, et chacune des matrices MicroLED (28) à l'intérieur du dispositif d'éclairage (20) est adressable individuellement ou en groupe par un contrôleur (32) pour illuminer la pluralité de matrices MicroLED (28) dans un ou plusieurs motifs différents pour apparaître sous la forme de l'objet tridimensionnel (22).

2. Dispositif d'éclairage (20) selon la revendication 1, dans lequel chacune des matrices MicroLED (28) mesure 0,2 mm x 0,2 mm.

3. Dispositif d'éclairage (20) selon la revendication 1, dans lequel la pluralité de matrices MicroLED (28) définissent un pas centre-à-centre supérieur à 0,3 mm entre des matrices MicroLED (28) adjacentes à l'intérieur de la pluralité de matrices MicroLED (28).

4. Dispositif d'éclairage (20) selon la revendication 1, dans lequel la pluralité de matrices MicroLED (28) définissent un pas centre-à-centre de 1 mm à 6 mm entre des matrices MicroLED (28) adjacentes à l'intérieur de la pluralité de matrices MicroLED (28).

5. Dispositif d'éclairage (20) selon la revendication 1, comprenant en outre une couche diffusante recouvrant la pluralité de matrices MicroLED (28).

6. Dispositif d'éclairage (20) selon la revendication 1, dans lequel chaque matrice MicroLED à l'intérieur de la pluralité de matrices MicroLED (28) est adressable individuellement par le contrôleur (32) ;
dans lequel chaque matrice MicroLED à l'intérieur de la pluralité de matrices MicroLED (28) est configurée pour avoir au moins un paramètre parmi une brillance ou une couleur qui est ajustable en réponse à un ordre par le contrôleur (32) ; et
dans lequel le contrôleur (32) est configuré pour illuminer la pluralité de matrices MicroLED (28) pour lui donner l'aspect de l'objet tridimensionnel (22).

7. Dispositif d'éclairage (20) selon la revendication 1,
dans lequel chaque matrice MicroLED à l'intérieur de la pluralité de matrices MicroLED (28) est configurée pour avoir au moins un paramètre parmi une brillance ou une couleur qui est ajustable en réponse à un ordre par le contrôleur (32).

8. Dispositif d'éclairage (20) selon la revendication 1, dans lequel le contrôleur (32) est configuré pour amener la pluralité de matrices MicroLED (28) à s'illuminer en réponse à un signal de commande (34).

9. Dispositif d'éclairage (20) selon la revendication 8, dans lequel le signal de commande (34) correspond à un signal parmi un signal de virage ou une condition de freinage ou une condition d'inversion du véhicule ou une illumination d'avertissement ou de signalement affiché en utilisant un phare arrière ou un feu avant.

10. Dispositif d'éclairage (20) selon la revendication 1, dans lequel l'objet tridimensionnel est animé avec un motif à variation temporelle.

11. Dispositif d'éclairage (20) selon la revendication 10, dans lequel le motif à variation temporelle fait apparaître l'objet tridimensionnel (22) pour qu'il se déplace jusque dans un plan sur ou parallèlement à la carte à circuit imprimé (30).

12. Dispositif d'éclairage (20) selon la revendication 10, dans lequel le motif à variation temporelle fait apparaître l'objet tridimensionnel pour qu'il sorte d'un plan sur ou parallèlement à la carte à circuit imprimé (30).

13. Dispositif d'éclairage (20) selon la revendication 10, dans lequel le motif à variation temporelle fait apparaître l'objet tridimensionnel (22) pour qu'il augmente en taille dans le temps.

14. Dispositif d'éclairage (20) selon la revendication 10, dans lequel l'objet tridimensionnel (22) est animé avec un motif pulsatoire.

15. Dispositif d'éclairage (20) selon la revendication 1, comprenant en outre une source d'illumination discrète (27).
